(19) Europäisches Patentamt
European Patent Office
Office européen des brevets

(11) **EP 1 867 995 A1**

(12) **EUROPEAN PATENT APPLICATION**

(43) Date of publication:
**19.12.2007 Bulletin 2007/51**

(51) Int Cl.:
***G01N 33/574*** (2006.01)

(21) Application number: **06290988.2**

(22) Date of filing: **15.06.2006**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU** | (72) Inventors:<br>• **Darbouret, Bruno**<br>  **30330 Tresques (FR)**<br>• **Sarkissian, Gaiané**<br>  **30900 Nimes (FR)** |
| (71) Applicant: **Cézanne S.A.S.**<br>**30035 Nimes Cédex 1 (FR)** | (74) Representative: **Andrae, Steffen et al**<br>**Andrae Flach Haug**<br>**Balanstrasse 55**<br>**81541 München (DE)** |

(54) **In vitro method for diagnosing and monitoring metastasized bladder cancer using the determination of MMP-7 in the circulation of patients**

(57) Use of matrix metalloproteinase 7 (MMP-7) and/or its precursors and fragments with MMP-7 immunoreactivity measured in blood, serum or plasma samples obtained from a patient in diagnostic *in vitro* methods for the detection, early detection, monitoring and/or prognosis of metastatic bladder cancer in human patients.

**EP 1 867 995 A1**

**Description**

**1. Field of the invention**

The present invention relates to the use of circulating (serum) MMP-7 as a marker for the determination of bladder- cancer metastases (risk) and the early diagnostic of metastases in bladder cancer patients. The present invention relates also to the measurement of circulating MMP-7 for monitoring the evolution of bladder cancers as well as for measuring the efficiency of bladder cancer treatment.

**2. Background of the invention**

Bladder cancer

The generic term "bladder cancer" is used for urinary bladder cancers which represent a spectrum of diseases that can be allotted to three general disease groups: superficial, invasive, and metastatic. "Bladder cancer" is the fourth cancer in men and the eighth in women both in terms of incidence and mortality (Cohen SC et al, Urol. Clin. N. Am (1992) 19, 421-428; Boring CC et al, CA Cancer J. Clin (1994) 44, 7-26). More than 90% of bladder cancer cases are transitional cell carcinoma (TCC), approximately 5% are squamous cell carcinomas (SCC), and less than 2% are adenocarcinoma.

At diagnosis about 75% of patients have superficial bladder cancer and 25% have invasive (into the muscle layer) or metastatic (into the lymph nodes or other organs usually the lungs, bones or liver) disease. Although superficial bladder tumors tend to recur frequently, most of recurrent tumors stay in the superficial areas. However, 5-30% of superficial tumors progress to invasive disease. Invasive tumors have a higher risk to metastasize. For untreated metastatic disease, the 2-year survival rate is less than 5%.

Currently, the main diagnostic procedures for bladder cancer are the urine cytology and histopathological examination of biopsies from the bladder wall taken during cystoscopy and cystectomy respectively. Urine cytology is "the gold standard" with high specificity but lower sensitivity in good-differentiated tumors. As recurrences in bladder cancer are frequent (up to 70% of cases) patients need periodic cystoscopies (Stein et al, J Urol (1998) 160, 654-659). Cystoscopy is an expensive invasive method that creates discomfort to the patient. Therefore, there is a need for a diagnosis method less invasive than cystoscopy and more efficient than cytology, which could also be used for the follow-up of bladder tumors.

Stage and grade (TNM-stratification) of bladder cancer are currently the most useful tools for taking therapeutic decisions and evaluating the prognosis of bladder cancer patients. However, as there are remarkable differences in biological behavior and "biological potential" of tumors classified in the same stage, it is very difficult to predict which superficial tumors will recur and which tumors will give distant metastases (Syrigos KN et al, Hybrid Hybridomics (2004) 23 (6), 335-42).

Diagnosis of bladder cancer metastasis is largely based on a variety of imaging techniques including radiography, ultrasonography, computed tomography (CT) and magnetic resonance imaging (MRI). Ultrasonography is the simplest, most non-invasive, and cheapest, but relies on the skill of the operator. CT and MRI are also non-invasive, and the sensitivities of these techniques have recently been improved. However, these techniques are time consuming and costly.

Therefore, there is a need for a sensitive *in vitro* test for early diagnosis of metastases in patients with primary bladder cancer, using a sample of a biological fluid, more specially a blood, serum or plasma sample, obtained from the patient. A biological marker the pathophysiological levels of which in a biological fluid are significantly correlated with the metastatic forms of bladder cancer but not, or at least not with the same significance, with other forms of bladder cancer, would form a valuable aid in the evaluation of the extension of the disease. It may help to limit the use of invasive examination and to adapt therapeutics earlier.

However, most scientific work relating to potential biomarkers for bladder cancer has been carried out with tissue samples. Taken into account that cystoscopy/cystectomy are standard methods for monitoring and treating bladder cancer, and that the repeated surgical biopsy is an integral part of routine patient management, bladder tumor biopsies are rather readily available for pathological examination and for molecular biological examination of gene expression on the transcript (mRNA) level. The aim of molecular biological studies is to identify constitutively expressed sequences of nucleic acids and sequences whose expression is altered during disease processes. For bladder cancer, results of an extensive study of altered gene expression are reported in US 6,936,417 B2. Further results of such study, using high throughput microarray technology, are reported in US 2004/0076955 A1. It is, however, well-known that altered gene expression patterns, for example disease related altered occurrences of transcripts (RNA) in tumor cells, are not necessarily and not predictably reflected on the protein level. The assumption that there is a "one-to-one" relation between a measured mRNA ratio and the associated protein ratio has been shown to be invalid (see, for example, Amit Mehra et al., Insights into the Relation Between mRNA and Protein Expression Patterns: I. Theoretical Considerations, Biotechnol. Bioeng. 2003, 84, 822-833; Clemens, M.J., Bommer U.A., "Translational control: the cancer connection", Int. J. Biochem. Cell Biol. 1999, 31, 1-23). In other words, increased expression on the transcript level does not mean that also subsequent step as translation, a possibly required posttranslational modification and a secretion will take place and that the corresponding protein is finally found also in the corresponding tissue or, in secreted form, even in a surrounding biological fluid. Further unpredictable factors, which destroy a one-to-one correlation between an mRNA and the level of its translational product (protein) in a biological fluid are e.g. the potential lack of stability of a secreted protein in the biological fluid, and the rate of its clearance by degradation or binding to associated receptors and other physiological binders and substrates.

Based on molecular biological studies, a large number of molecules have been identified as being differentially expressed in bladder cancer and possibly involved in carcinogenic pathophysiological mechanisms, and some of them have been studied in more detail as potential markers for the diagnosis, recurrence, and prognosis of bladder cancer. Because urine is the body liquid in most direct contact with all forms of bladder cancer, most studies aimed at the determination of potential biomarker molecules in urine.

The usefulness of urine assays of selected biomarkers (especially bladder tumor antigen [BTA-Stat, BTA-TRAK], nuclear matrix protein [NMP-22], fibrin/fibrinogen degradation products [FDP]) for the detection and surveillance of TCC is being intensely studied at present. These tests have, however, high false-positive and false-negative rates. Clinical studies are in progress for the assessment of the other newer assays based on telomerase and microsatellite analysis (Jichlinski P, Curr Opin Urol. (2003) 13(5), 351-355 ; Simon MA et al, Crit Rev Oncol Hematol. (2003) 47(2), 91-107).

However, at this time, neither of the urinary assays investigated so far have been shown to have the potential to reliably replace cystoscopy and imaging techniques for the diagnosis and surveillance of bladder cancer effectively.

Matrix Metalloproteinases

Among biomolecules the expression of which is found altered in various forms of bladder cancer are matrix metalloproteinases (MMP) and tissue inhibitors (TIMP) which affect the activity of said metalloproteinases (see SUMI T ET AL: "Expression of matrix metalloproteinases in human transitional cell carcinoma of the urinary bladder", Oncol Rep. 2003, Mar-Apr; 10(2);345-349; FURUKAWA A ET AL: "Role of the matrix metalloproteinase and tissue inhibitors of metalloproteinase families in noninvasive and invasive tumors transplanted in mice with severe combined immunodeficiency", Urology 1998, May; 51 (5) :849-853).

Matrix metalloproteinases form a group of functionally and structurally related molecules which, in addition to trivial names, are characterized by systematic abbreviations consisting of the letters MMP and a number (i.e. MMP-xx). In humans, 16 members of this family have been identified (Kleiner et al., (1999) Cancer Chemother Pharmacol., 1999; 43 Suppl: S42-15), and if non-human species are considered as well, the number of MMPs is still higher (a minireview by Hideaki Nagase et al. (1999), J. Biol. Chem. 274, pp. 21491-21494 lists 20 family members).

Matrix metalloproteinases (MMPs), also called matrixins, constitute a family of zinc and calcium dependent endopeptidases that function in the breakdown of extracellular matrix (ECM).

The MMPs have been implicated in a wide variety of normal physiological processes including bone and tissue remodelling, uterine resorption, trophoblast implantation, angiogenesis, embryonic development and normal wound healing (Kleiner and Stetler-Stevenson, Cancer Chemother Pharmacol. (1999) 43 Suppl:S42-51; Nagase, H. et al (1999) J. Biol. Chem. 274, 2191). When present in excess, they are also thought to participate in the accelerated breakdown of ECM that is associated with a number of diseases including periodontitis (Reynolds and Meikle, J R Coll Surg Edinb. (1997) 42 (3) :154-60), arthritis, atherosclerosis, tissue ulcerations, tumor cell invasion, and metastasis (Birkedal-Hansen et al., Crit Rev Oral Biol Med. (1993) 4(2):197-250).

Matrix metalloproteases are known to play an important role in tumor invasion by mediating degradation of extracellular matrix (Shiomi T et al, Cancer Metastasis Rev. (2003) 22(2-3), 145-152).

For example, metalloproteinases of the types MMP-2 and MMP-9 were reported to be elevated in renal cell carcinoma (RCC) tumor tissue, and their expression levels were suggested to correlate with tumor aggressiveness (Kugler et al, J Urol. 1998 Nov; 160 (5) : 1914-8).

Lein M et al found that MMP-9 was elevated in tumor tissue of patients with RCC, whereas MMP-2 was not different between tumor tissue and normal counterparts (Int J Cancer. 2000 Mar 15;85(6):801-4).

Sherief et al described that it is possible to detect in urine from patients with renal cell carcinoma an elevated enzyme activity of matrix metalloproteinases, which leads to the degradation of normally excreted extracellular matrix (ECM) proteins. Decreased urinary ECM proteins were analysed by SDS-PAGE followed by immunoblotting with antibodies against three types of ECM proteins. Enzyme matrix metalloproteinase activity was measured using fluorescein conjugated collagen IV substrate (Sherief MH, J Urol. (2003) 169 (4), 1530-4).

In an enzymatic determination, MMPs are not directly measured. Since in such measurements only an enzymatic activity is determined, they do not allow it to draw reliable conclusions regarding the type of MMP(s) responsible for the observed enzymatic activity.

Results of other studies dealing with the pathophysiological role of matrix metalloproteinases are reported, for example, in publications as ICHIKAWA Y ET AL: "Detection of regional lymph node metastases in colon cancer by using RT-PCR for matrix metalloproteinase 7, matrilysin", Clin Exp Metastasis. 1998 Jan; 16(1):3-8; MASAKI T ET AL: "Matrilysin (MMP-7) as a significant determinant of malignant potential of early invasive colorectal carcinomas", British Journal of Cancer (2001) 84, 1317-1321; KUEFER R ET AL: "The role of an 80 kDa fragment of E-cadherin in the metastatic progression of prostate cancer", Clin Cancer Res. 2003 Dec 15; 9 (17) :6447-6452; or MEADE-TOLLIN LC ET AL: "Differential expression of matrix metalloproteinases in activated c-ras-Ha-transfected immortalized human keratinocytes", Br J Cancer. 1998 Mar;77(5):724-30.

Among the matrix metalloproteinases the expression of which has been studied there also is MMP-7 (matrilysin), which is discussed also with respect to bladder cancer tissues (see e.g. SUMI T ET AL; supra; and FURUKAWA A ET AL; *supra).*

SUMI ET AL; *supra,* report results of a study where the expression of MMP-7 is evaluated on transitional cell carcinoma (TCC) tissue samples using immunostaining with a specific antibody. MMP-7 was expressed in TCC, having a tendency to be more strongly expressed in high than in low grade tumors. However, there was no significant difference of MMP-7 expression between advanced and low stages. The authors suggested that the increased expression of MMP-7 in high grade TCC tissues of the urinary bladder may be associated with tumor invasion and metastasis.

FURUKAWA ET AL; supra, report significantly higher gene expression of MMP-2 in the invasive bladder tumor line than in the noninvasive tumor line. However, a MMP-7 (matrilysin) expression was not detected in either of the lines.

In US 6,936,417 B2 expression of MMP-7 (matrilysin) was not correlated with invasive TCC and is not mentioned as invasive TCC-related gene. In addition, the transcript level of the matrilysin gene was not increased during progression of bladder cancer when comparing three different biopsy pools representing the transition from normal urothelium to superficial tumor, and further on to invasive TCC.

The observation that the expression of a number of matrix metalloproteinases is altered in various cancer tissues has prompted the group of Stanley Zucker et al. to examine MMP levels also in the plasma of patients with different forms of cancer: ZUCKER S ET AL: "Measurement of matrix metalloproteinases and tissue inhibitors of metalloproteinases in blood and tissues. Clinical and experimental applications", Ann NY Acad Sci. 1999 June 30; 878: 212-27; and ZUCKER S ET AL: "Measurement of matrix metalloproteinases (MMPs) and tissue inhibitors of metalloproteinases (TIMP) in blood and urine: potential clinical applications", Adv Clin Chem. 2004;38:37-85.

The authors, in the first of the two papers mentioned, evaluated matrix metalloproteinase (MMPs) levels in the plasma of patients with cancer. They report, inter alia, increased plasma levels of MMP-7 (matrilysin) in patients with bladder cancer. The pathologic stage and tumor grade of patients are not specified in the document. In addition, the clinical usefulness of the determination of MMP-7 assay in the plasma of patients with bladder cancer is questioned. In view of the fact that matrilysin (MMP-7) is present already early in tumor development, i.e. at a time when tumors are not known to be invasive or metastatic, the authors suggest that matrilysin may not be involved in metastasis and MMP-7 does not represent a valuable marker for diagnosis and prognosis of metastasized bladder cancer.

In the second of the mentioned publications the authors provide a comprehensive review on potential future developments and applications of the measurement of MMPs and tissue inhibitors of metalloproteinases (TIMPs) in blood and urine. They display a table of metalloproteinases as being possibly useful for diagnosis and prognosis of bladder cancer, but among the list of metalloproteinases they reject MMP-7 as being a candidate for diagnosis of metastasized bladder cancer.

Regarding the importance of MMP-7 in cancer progression, there are conflicting reports.

Some studies suggested a possible correlation between overexpression in tissue of MMP-7 and metastatic events in carcinoma of the bladder and colon.

On the other hand, it has been reported that the transcript level of matrilysin gene in invasive bladder TCC is comparable with that of normal urothelium and superficial tumor (US 6,936,417 B2). Similarly, Meade-Tollin et al., supra, described an increased secretion of matrilysin in both benign and malignant tumorigenic cells. Kuefer et al., supra, reported that matrilysin is significantly underexpressed in metastases compared with localized prostate cancer.

Therefore, the relationship between the expression of MMP-7 and its potential role in the malignant behavior of tumors remains controversial.

When the inventors, within the framework of a more extensive study on the role and significance of MMP-7 (matrilysin) in cancers of the urogenital tract, found a clear and diagnostically significant correlation of the levels of MMP-7 in the circulation (blood, serum, plasma) with a metastatic form of bladder cancer, in clear contrast to other forms of bladder cancer, such findings were considered very surprising and not precedented by the prior art as discussed above.

The findings on which the present invention is based suggest that circulating matrilysin levels constitute a parameter which makes it possible to overcome at least some of the deficiencies of prior art diagnostic methods discussed above and can be used, with high significance, as a predictor or detector of metastasis in a bladder cancer patient.

Similarly, circulating MMP-7 levels may be used to detect an invasive tumor in a bladder cancer patient and to monitor evolution of bladder cancer.

Consequently, according to claim 1 the present invention provides the use of circulating matrix metalloproteinase 7 (MMP-7) and/or its precursors and fragments with MMP-7 immunoreactivity, optionally in combination with the determination/evaluation of at least one further established tumor marker according to claim 10, as biomarkers for the detection, early detection, monitoring and/or prognosis of metastatic bladder cancer in human patients.

Further, the invention provides, according to claim 2, a diagnostic in vitro method for diagnosing or monitoring metastatic bladder cancer in a human patient by determining in a sample from the circulation of the patient a biomarker the level of which is indicative for the presence and/or severity of metastatic bladder cancer, and associating the level of the measured biomarker with the presence or severity of metastatic blader cancer, wherein the measured biomarker is MMP-7 which is determined directly as measurable MMP-7 immunoreactivity.

Some suitable or preferred embodiments of such method are recited in claims 3 to 9.

As is explained in more detail in the following experimental section, the inventors have demonstrated that in a population of 38 bladder cancer patients with no identified metastasis the MMP-7 level of only a small proportion (18.4%) is above a cut-off. On the other hand, using in the present instance the same cut-off, there is a clear correlation between circulating MMP-7 and metastasis in bladder cancer patients.

Based on the results reported in the present application, MMP-7 is a candidate for a novel serum/plasma marker allowing the detection of bladder cancer metastases, notably in order to adapt the cancer treatment to the patient.

Measuring of MMP-7 levels in a bladder cancer patient can be useful and helpful in monitoring the evolution and follow-up of bladder cancer as well as for measuring the efficiency of a cancer treatment.

The present invention therefore provides, in one of its aspects, an *in vitro* method of diagnosing or detecting metastasis in a bladder cancer patient comprising periodically analyzing a sample of blood and/or serum and/or plasma from such patient for MMP-7; comparing the MMP-7 levels in such sample with levels of MMP-7 in preferably the same sample type of a normal human control or non-metastatic bladder cancer patient sample, wherein an increase in MMP-7 levels in the patient versus the normal human control or non metastatic bladder cancer patient is associated with a bladder cancer which has metastasized.

In case of increased MMP-7 levels in the patient with primary bladder cancer said method is used to measure the efficacy of adjuvant treatment, comprising carrying out periodic in vitro tests by determining the MMP-7 and/or pro-MMP-7 concentration in a sample of blood and/or serum and/or plasma from a bladder cancer patient, wherein serum concentration of MMP-7 and/or pro-MMP-7 coming back to a basal level is indicative of treatment efficacy.

In another of its aspects, the method is also used to measure the efficacy of treatment for bladder cancer patients having metastases, comprising carrying out periodic *in vitro* tests by determining the MMP-7 and/or pro-MMP-7 concentration in a sample of blood and/or serum and/or plasma from a bladder cancer patient, wherein a decreased level of MMP-7 and/or pro-MMP-7 is indicative of sensitivity or success of treatment.

The method can also be used for detecting an invasive tumor in a patient with bladder cancer, comprising carrying out periodic *in vitro* tests, each test comprising determining the MMP-7 concentration in a sample of blood or a serum and/or plasma from a patient presenting superficial bladder cancer, wherein an increase in MMP-7 concentration between tests indicates a tumor which has become invasive in the patient.

According to another aspect the present invention encompasses the determination of MMP-7, precursors of MMP-7 and/or fragments of MMP-7 with MMP-7 immunoreactivity in the circulation of a patient suspected to have bladder cancer as co-marker in combination with the determination of at least one further established tumor marker, using a computer programm for statistical analysis of more than one measurement of biomarkers potentially relevant for the diagnosis of metastatic bladder cancer. By way of example only, said at least one further established tumor marker may be selected from the group consisting of CEA, AFP, TPA, CYFRA 21-1, CA 19-9, CA 125, CA 72-4 (TAG-72), CA 15-3, CA 549, hCG, MCA, NSE, PSA, SCC, LDH (lactate dehydrogenase) and PAL (alkaline phosphatase).

The invention will now, for illustrative purposes only, further explained on the basis of clinical measurements and figures.

Description of the drawings

Figure 1 is a standard curve for the measurement of MMP-7 levels in serum of patients with bladder cancer with an assay as described in the experimental section;

Figure 2 is a diagrammatic representation of the results of measurements of MMP-7 in sera of patients with primary bladder cancer with no identified metastasis, patients with metastasized bladder cancer and healthy controls;

Figure 3a is a ROC (Receiver-Operating Characteristics) curve indicative for the clinical diagnostic validity (sensitivity vs. specificity) of the determination of increased MMP-7 levels in sera of patients with metastasized bladder cancer;

Figure 3b is a ROC (Receiver-Operating Characteristics) curve indicative the poor diagnostic value (sensitivity vs. specificity) of the determination of serum MMP-7 levels for detection of primary bladder cancer with no identified metastasis;

**Experimental section and Examples**

Materials and methods

Serum samples

All serum samples were isolated from venous human blood samples taken from either patients diagnosed with primary bladder carcinoma (n=38), from patients diagnosed with metastasized bladder carcinoma (n=60), or from normal volunteer donors (n=111) (after informed consent was given from each individual).

Immunoassay

The assay was performed using Human MMP-7 Quantikine ELISA Kit (DMP700) (R&D Systems Europe Ltd. Abingdon UK), according to R&D Systems protocol.
The assay employs the quantitative sandwich enzyme immunoassay technique. A monoclonal antibody specific for MMP-7 has been pre-coated onto a microplate. Standards and samples are pipetted into the wells, and MMP-7 is bound by the immobilized antibody. After washing away unbound substances, an enzyme-linked polyclonal antibody specific for MMP-7 is added to the wells. Following a wash to remove unbound antibody-enzyme reagent, a substrate solution is added to the wells and color develops in proportion to the amount of total MMP-7 (pro and/or active) bound in the initial step. The color development is stopped and the intensity of the color is measured at 450 nm wavelength absorbance by FLUOstar microplate reader (BMG Labtechnologies, GmbH, Offenburg, Germany).
The assay was performed according to the manufacturer R&D Systems' prescribed assay protocol.

Measurements and results

a) Standard curve and measurement of MMP-7 levels in serum of patients with bladder cancer (BC)

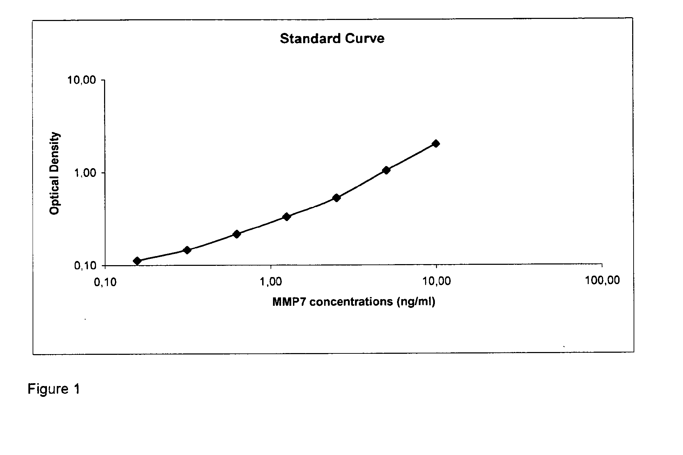

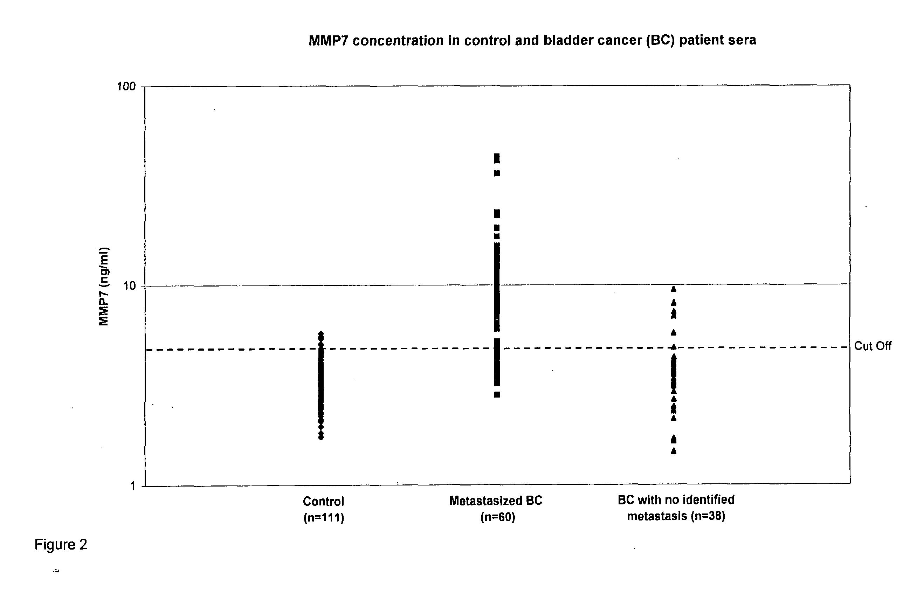

The standard curve obtained using the recombinant human MMP-7 (Quantikine kit standards). The recombinant human MMP-7 was diluted in calibrator diluent RD6-28 (Quantikine kit) to give MMP-7 standard values of 0.156, 0.312, 0.625, 1.25, 2.5, 5, and 10ng/ml.
Samples with >10ng/ml of MMP-7 concentration were diluted with calibrator diluent RD6-28 (Quantikine kit) to produce samples with values within dynamic range of the assay.
Standards or samples (50$\mu$L) and assay diluent (100$\mu$l) were added to the wells of the microtiter plate. After incubation for 2 hours at room temperature on a horizontal orbital shaker, each well was washed 4 times with wash buffer (400$\mu$l). 200$\mu$l of MMP-7 conjugate were added to each well, followed by incubation of microtiter plate for 2 hours at room temperature on the shaker. After 4 washing steps and addition of substrate solution (200$\mu$l) to each well, microplate was incubated for 30 minutes at room temperature. The reaction was stopped with stop solution (50 $\mu$l) and the optical density of each well was determined using FLUOstar microplate reader to 450nm.
Normal human sera (n=111) were used as control samples.
The standard curve is shown in Figure 1.
Patients with metastasized bladder cancer (BC meta) had significantly increased levels of serum MMP-7 as compared to primary bladder cancer patients with no identified metastasis (BC) and normal controls (Table 1 and Figure 2).

Table 1

| Groups | n | Mean $\pm$ SD | Sensitivity (cut-off 4.78 ng/ml) | Specificity (cut-off 4.78 ng/ml) |
|--------|-----|------------------|----------------------------------|----------------------------------|
| BC meta | 60 | 10.44 $\pm$ 8.5 ng/ml | 75% | 94.6% |
| BC | 38 | 4.57 $\pm$ 4.97 ng/ml | 18.4% | 94.6% |
| Control | 111 | 3.29 $\pm$ 0.83 ng/ml | | |

In Table 1:

$$Sensitivity = True\text{-}positive/True\text{-}positive + False\text{-}negative;$$

$$Specificity = True\text{-}negative/\ True\text{-}negative + False\text{-}positive$$

b) Statistical analysis and ROC curve

The clinical sensitivity versus clinical specificity diagram is presented by ROC (Receiver-Operating Characteristics) curve.

For calculations of clinical sensitivity and clinical specificity, 95% confidence intervals were determined based on the binomial distribution (Besnard et Morin, In: Immunostat, Paris, Edition Nucléon, 227-251 (1997)).

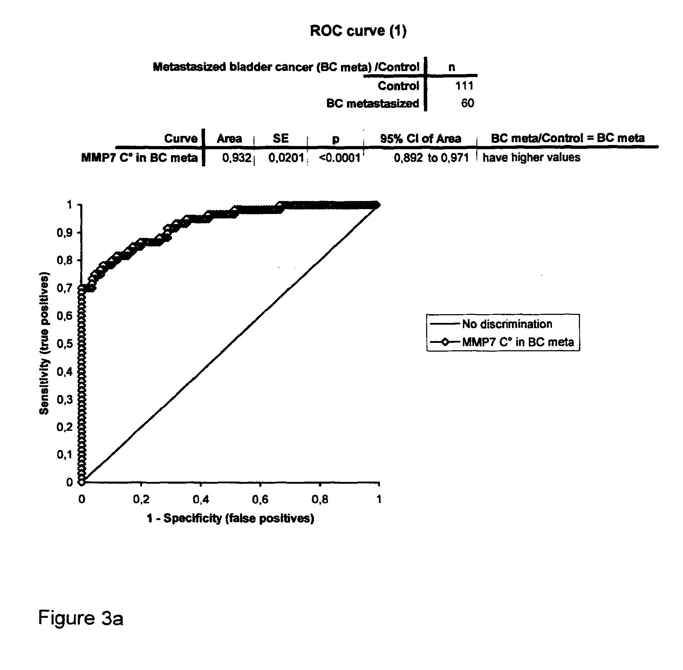

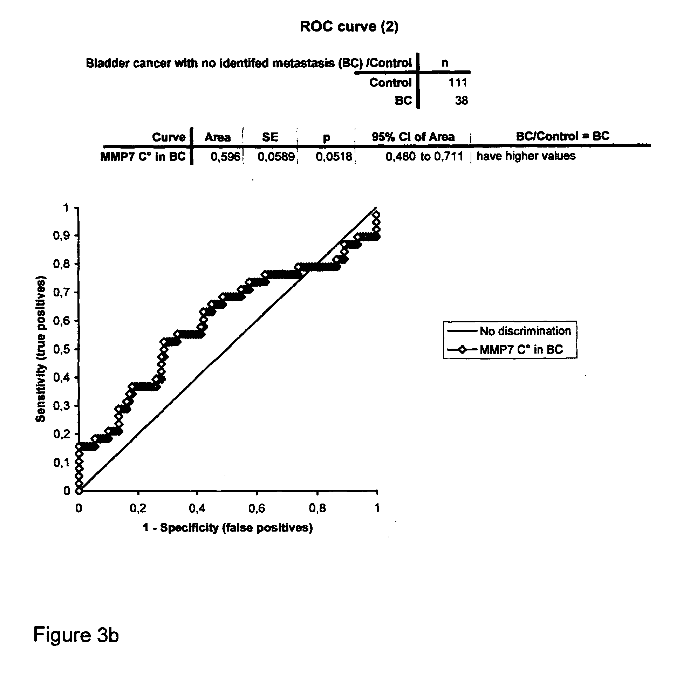

Employing a cut-off value of 4.78 ng/ml, the serum MMP-7 concentration had a sensitivity of 75% and specificity of 94.6% for the discrimination between metastasized bladder cancer and healthy subjects (Table 1 and Figure 3a). However, using the same cut-off value, the clinical sensitivity of the MMP-7 determination in primary bladder cancer with no identified metastasis is only 18.4% (Table 1 and Figure 3b).

AUC (Area under curve) values calculated by ROC analysis are 0.932 and 0.596 for metastasized bladder cancer and primary bladder cancer with no identified metastasis, respectively (Figure 3a and 3b). A test with AUC of 0.932 is accurate, whereas one with an AUC of 0.596 is performing no better than chance.

These data prove that circulating MMP-7 (MMP-7 in serum or plasma) can be used, optionally in combination with other tumor markers or other clinical parameters, as a marker for the determination (detection) of bladder cancer metastases (risk) and the early diagnostic of metastases in bladder cancer patients.

**Claims**

1. Use of circulating matrix metalloproteinase 7 (MMP-7) and/or its precursors (pro-MMP-7) and fragments with MMP-7 immunoreactivity as biomarkers for the detection, early detection, monitoring and/or prognosis of metastatic bladder cancer in human patients.

2. Diagnostic *in vitro* method for diagnosing or monitoring metastatic bladder cancer in a human patient by determining in a sample from the circulation of the patient a biomarker the level of which is indicative for the presence and/or severity of metastatic bladder cancer, and associating the level of the measured biomarker with the presence or severity of metastatic blader cancer, wherein the measured biomarker is MMP-7 which is determined directly as measurable MMP-7 immunoreactivity.

3. Method according to claim 2, which is conducted as method for monitoring the evolution of metastatic bladder cancer in an bladder cancer patient, comprising measuring in a serum or plasma sample obtained from said patient the levels of MMP-7 immunoreactivity at selected consecutive time intervals, wherein a variation in the measured levels of MMP-7 over time is indicative of the evolution of metastatic bladder cancer.

4. Method according to claim 2, which is conducted as method for measuring the efficacy of a treatment of metastatic bladder cancer, comprising measuring in a serum or plasma sample obtained from said patient the levels of MMP-7 immunoreactivity at selected consecutive time intervals, wherein a decrease in the measured levels of MMP-7 between the measurements is indicative of the efficacy of the treatment.

5. The method according to any one of claims 2 to 4 wherein MMP-7 immunoreactivity is selectively measured using specific ligand assay techniques.

6. The method according to claim 5 wherein MMP-7 immunoreactivity is measured with an assay using at least one specific ligand that specifically binds to MMP-7.

7. The method of any one of claims 2 to 6, wherein the measured MMP-7 immunoreactivity can be attributed to the presence of MMP-7 itself and/or of its proenzyme form (pro-MMP-7) and/or MMP-7 fragments displaying MMP-7 immunoreactivity.

8. The method according to any of claims 2 to 7, wherein the result of the measurement of MMP-7 and MMP-7 immunoreactivity respectively, for diagnostic purposes, is combined with the results of the measurement of at least one further established tumor marker, using a computer programm for statistical analysis of more than one measurement of biomarkers potentially relevant for the diagnosis of metastatic bladder cancer.

9. The method according to claim 8, wherein the at least one further established tumor marker is selected from the group consisting of CEA, AFP, TPA, CYFRA 21-1, CA 19-9, CA 125, CA 72-4 (TAG-72), CA 15-3, CA 549, hCG, MCA, NSE, PSA, SCC, LDH, PAL.

10. Use of circulating matrix metalloproteinase 7 (MMP-7) and/or its precursors (pro-MMP-7) and fragments with MMP-7 immunoreactivity as co-biomarkers in combination with established tumor markers for the detection, early detection, monitoring and/or prognosis of metastatic bladder cancer in human patients.

Figure 1

MMP7 concentration in control and bladder cancer (BC) patient sera

Figure 2

## ROC curve (1)

| Metastasized bladder cancer (BC meta) /Control | n |
|---|---|
| Control | 111 |
| BC metastasized | 60 |

| Curve | Area | SE | p | 95% CI of Area | BC meta/Control = BC meta |
|---|---|---|---|---|---|
| MMP7 C° in BC meta | 0,932 | 0,0201 | <0.0001 | 0,892 to 0,971 | have higher values |

Figure 3a

## ROC curve (2)

| Bladder cancer with no identifed metastasis (BC) /Control | n |
|---|---|
| Control | 111 |
| BC | 38 |

| Curve | Area | SE | p | 95% CI of Area | BC/Control = BC |
|---|---|---|---|---|---|
| MMP7 C° in BC | 0,596 | 0,0589 | 0,0518 | 0,480 to 0,711 | have higher values |

Legend:
— No discrimination
—◇— MMP7 C° in BC

Figure 3b

European Patent
Office

**EUROPEAN SEARCH REPORT**

Application Number

EP 06 29 0988

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/043121 A (VITATEX, INC.) 12 May 2005 (2005-05-12) * abstract * * paragraphs [0004], [0028] * * example 12 * * claims 12-23,32-35 * ----- | 1-10 | INV. G01N33/574 |
| X | KIM J.H. ET AL.: "Alterations in transcription clusters underlie development of bladder cancer along papillary and nonpapillary pathways." LABORATORY INVESTIGATION, vol. 85, 2005, pages 532-549, XP002400517 * abstract * * page 532, column 2, last paragraph - page 533, paragraph 1 * * page 534, column 1, last paragraph - column 2 * * page 536, column 1 - column 2, paragraph 1 * * page 538 * * page 540 - page 542, column 1, paragraph 1 * ----- | 1-10 | |
| X,D | SUMI T. ET AL.: "Expression of matrix metalloproteinases in human transitional cell carcinoma of the urinary bladder." ONCOL. REP., vol. 10, no. 2, March 2003 (2003-03), pages 345-349, XP009072765 ISSN: 1021-335X * abstract * * page 348, column 1 * ----- -/-- | 1-10 | TECHNICAL FIELDS SEARCHED (IPC) G01N C12N |

The present search report has been drawn up for all claims

| Place of search | Date of completion of the search | Examiner |
|---|---|---|
| Munich | 26 September 2006 | Giry, Murielle |

EPO FORM 1503 03.82 (P04C01)

**European Patent Office**

**EUROPEAN SEARCH REPORT**

Application Number

EP 06 29 0988

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AHN K S ET AL: "Increasing expression level of Ets-1 via HIF-1alpha induces the angiogenesis and invasiveness human bladder cancer: It may be one of useful molecular markers to determine the progression of human bladder cancer." EUROPEAN UROLOGY SUPPLEMENTS, ABSTRACT 211, vol. 3, no. 2, February 2004 (2004-02), page 55, XP002400535 & 19TH CONGRESS OF THE EUROPEAN ASSOCIATION OF UROLOGY; VIENNA, AUSTRIA; MARCH 24-27, 2004 ISSN: 1569-9056 * abstract * | 1-10 | |
| X | SABHA N. ET AL.: "Matrix metalloproteinase-7 and epidermal growth factor receptor mediate hypoxia-induced extracellular signal-regulated kinase 1/2 mitogen-activated protein kinase activation and subsequent proliferation in bladder smooth muscle cells." IN VITRO CELL. DEV. BIOL. - ANIMAL, vol. 42, no. 5-6, May 2006 (2006-05), pages 124-133, XP009072784 ISSN: 1071-2690 * abstract * * page 127, column 2, last paragraph - page 129, column 1, paragraph 1 * * figure 6 * | 1-10 | |

-----

-/--

| TECHNICAL FIELDS SEARCHED (IPC) |
|---|

The present search report has been drawn up for all claims

| Place of search | Date of completion of the search | Examiner |
|---|---|---|
| Munich | 26 September 2006 | Giry, Murielle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

**EUROPEAN SEARCH REPORT**

Application Number

EP 06 29 0988

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GOTANDA T. ET AL.: "Molecular basis for the involvement of thymidine phosphorylase in cancer invasion." INT. J. MOL. MED., vol. 17, no. 6, June 2006 (2006-06), pages 1085-1091, XP009072798 ISSN: 1107-3756 * abstract * * page 1089, column 1 * * page 1090, column 1, last paragraph - column 2, last paragraph * | 1-10 | |
| X | WALLARD M.J. ET AL.: "Comprehensive profiling and localisation of the matrix metalloproteinases in urothelial carcinoma." BRIT. J. CANCER, vol. 94, 27 February 2006 (2006-02-27), pages 569-577, XP002400518 * abstract * * page 571, column 1, paragraph 4; table 1 * | 1-10 | |
| A | PATENT ABSTRACTS OF JAPAN vol. 1996, no. 12, 26 December 1996 (1996-12-26) & JP 08 217800 A (FUJI YAKUHIN KOGYO KK), 27 August 1996 (1996-08-27) * abstract * | 1-10 | TECHNICAL FIELDS SEARCHED (IPC) |
| A | PATENT ABSTRACTS OF JAPAN vol. 2003, no. 12, 5 December 2003 (2003-12-05) & JP 2003 321494 A (DAIICHI FINE CHEMICAL CO LTD), 11 November 2003 (2003-11-11) * abstract * | 1-10 | |

The present search report has been drawn up for all claims

| Place of search | Date of completion of the search | Examiner |
|---|---|---|
| Munich | 26 September 2006 | Giry, Murielle |

**ANNEX TO THE EUROPEAN SEARCH REPORT
ON EUROPEAN PATENT APPLICATION NO.**

EP 06 29 0988

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2006

| Patent document cited in search report | | Publication date | Patent family member(s) | | Publication date |
|---|---|---|---|---|---|
| WO 2005043121 | A | 12-05-2005 | AU | 2004286307 A1 | 12-05-2005 |
| | | | CA | 2544373 A1 | 12-05-2005 |
| | | | EP | 1706720 A2 | 04-10-2006 |
| JP 08217800 | A | 27-08-1996 | JP | 2949467 B2 | 13-09-1999 |
| JP 2003321494 | A | 11-11-2003 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6936417 B2

- US 20040076955 A1

**Non-patent literature cited in the description**

- **COHEN SC et al.** *Urol. Clin. N. Am,* 1992, vol. 19, 421-428
- **BORING CC et al.** *CA Cancer J. Clin,* 1994, vol. 44, 7-26
- **STEIN et al.** *J Urol,* 1998, vol. 160, 654-659
- **SYRIGOS KN et al.** *Hybrid Hybridomics,* 2004, vol. 23 (6), 335-42
- **AMIT MEHRA et al.** Insights into the Relation Between mRNA and Protein Expression Patterns: I. Theoretical Considerations. *Biotechnol. Bioeng.,* 2003, vol. 84, 822-833
- **CLEMENS, M.J. ; BOMMER U.A.** Translational control: the cancer connection. *Int. J. Biochem. Cell Biol.,* 1999, vol. 31, 1-23
- **JICHLINSKI P.** *Curr Opin Urol.,* 2003, vol. 13 (5), 351-355
- **SIMON MA et al.** *Crit Rev Oncol Hematol.,* 2003, vol. 47 (2), 91-107
- **SUMI T et al.** Expression of matrix metalloproteinases in human transitional cell carcinoma of the urinary bladder. *Oncol Rep.,* March 2003, vol. 10 (2), 345-349
- **FURUKAWA A et al.** Role of the matrix metalloproteinase and tissue inhibitors of metalloproteinase families in noninvasive and invasive tumors transplanted in mice with severe combined immunodeficiency. *Urology,* May 1998, vol. 51 (5), 849-853
- **KLEINER et al.** *Cancer Chemother Pharmacol.,* 1999, vol. 43, 42-15
- **HIDEAKI NAGASE et al.** *J. Biol. Chem.,* 1999, vol. 274, 21491-21494
- **KLEINER ; STETLER-STEVENSON.** *Cancer Chemother Pharmacol.,* 1999, vol. 43, S42-51
- **NAGASE, H. et al.** *J. Biol. Chem.,* 1999, vol. 274, 2191
- **REYNOLDS ; MEIKLE.** *J R Coll Surg Edinb.,* 1997, vol. 42 (3), 154-60
- **BIRKEDAL-HANSEN et al.** *Crit Rev Oral Biol Med.,* 1993, vol. 4 (2), 197-250

- **SHIOMI T et al.** *Cancer Metastasis Rev.,* 2003, vol. 22 (2-3), 145-152
- **KUGLER et al.** *J Urol.,* November 1998, vol. 160 (5), 1914-8
- **LEIN M et al.** MMP-9 was elevated in tumor tissue of patients with RCC, whereas MMP-2 was not different between tumor tissue and normal counterparts. *Int J Cancer.,* 15 March 2000, vol. 85 (6), 801-4
- **SHERIEF MH.** *J Urol.,* 2003, vol. 169 (4), 1530-4
- **ICHIKAWA Y et al.** Detection of regional lymph node metastases in colon cancer by using RT-PCR for matrix metalloproteinase 7, matrilysin. *Clin Exp Metastasis,* January 1998, vol. 16 (1), 3-8
- **MASAKI T et al.** Matrilysin (MMP-7) as a significant determinant of malignant potential of early invasive colorectal carcinomas. *British Journal of Cancer,* 2001, vol. 84, 1317-1321
- **KUEFER R et al.** The role of an 80 kDa fragment of E-cadherin in the metastatic progression of prostate cancer. *Clin Cancer Res.,* 15 December 2003, vol. 9 (17), 6447-6452
- **MEADE-TOLLIN LC et al.** Differential expression of matrix metalloproteinases in activated c-ras-Ha-transfected immortalized human keratinocytes. *Br J Cancer.,* March 1998, vol. 77 (5), 724-30
- **ZUCKER S et al.** Measurement of matrix metalloproteinases and tissue inhibitors of metalloproteinases in blood and tissues. Clinical and experimental applications. *Ann NY Acad Sci.,* 30 June 1999, vol. 878, 212-27
- **ZUCKER S et al.** Measurement of matrix metalloproteinases (MMPs) and tissue inhibitors of metalloproteinases (TIMP) in blood and urine: potential clinical applications. *Adv Clin Chem.,* 2004, vol. 38, 37-85
- **BESNARD ; MORIN.** Immunostat. 1997, 227-251